# EUROPEAN PATENT APPLICATION

(11) **EP 4 039 252 A1**
(43) Date of publication of application: **10.08.2022**
(21) Application number: 22155377.9
(22) Date of filing: 07.02.2022
(51) Int. Cl.: A61K 9/20, A61K 31/485, A61K 9/28

(54) **A FILM COATED TABLET COMPRISING NALTREXONE HYDROCHLORIDE**

(30) Priority: 09.02.2021 TR 202101817
(71) Applicant: SANOVEL ILAC SANAYI VE TICARET A.S., Istanbul 34460 (TR)
(72) Inventor: OZDEN, Aydan, 34460 Istanbul (TR); PEHLIVAN AKALIN, Nur, 34460 Istanbul (TR); SUNEL, Fatih, 34460 Istanbul (TR)
(74) Representative: Sevinç, Erkan

(57) **Abstract**

The present invention relates to a film coated tablet comprising naltrexone hydrochloride and at least one pharmaceutically acceptable excipient wherein naltrexone hydrochloride has a d (0.9) particle size less than 50 µm. Furthermore, the present invention discloses a process for the preparation of a film coated tablet formulation comprising naltrexone hydrochloride. The process is simple, rapid, cost effective, time-saving and industrially convenient process.

## Description

### Field of the Invention

The present invention relates to a film coated tablet comprising naltrexone hydrochloride and at least one pharmaceutically acceptable excipient wherein naltrexone hydrochloride has a d (0.9) particle size less than 50 µm. Furthermore, the present invention discloses a process for the preparation of a film coated tablet formulation comprising naltrexone hydrochloride. The process is simple, rapid, cost effective, time-saving and industrially convenient process.

### Background of the Invention

The opioids are drugs that produce an analgesic and antinociceptive response with different degrees of potency depending on the pharmaceutical substance used. Naltrexone is an opioid antagonist which can inhibit pharmacological effects of both administered opioid agonists and endogenous agonists derived from the opioid system.

Naltrexone Hydrochloride is the hydrochloride salt of naltrexone, a noroxymorphone derivative with competitive opioid antagonistic activity. The chemical name of naltrexone hydrochloride is 17-(Cyclopropylmethyl)-4,5-alpha-epoxy-3,14-dihydroxy-morphinan-6-one hydrochloride and its chemical structure is shown in the Formula 1.

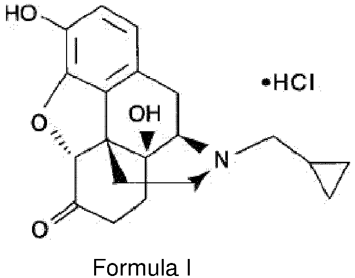

The compound and methods for the synthesis of naltrexone are described in U.S. Patent No. 3,332,950. When co-administered with morphine, heroin or other opioids on a chronic basis in a sufficient amount, naltrexone may reduce the incidence of physical dependence to opioids.

Naltrexone hydrochloride is commercially available in tablet form (Revia^{®}, DuPont) for the treatment of alcohol dependence and for the blockade of exogenously administered opioids.

Naltrexone hydrochloride is a poorly soluble drug. In the actual production of pharmaceutical composition, it often encounters problems of low dissolution or even unqualified.

Furthermore, one of the requirements for an acceptable pharmaceutical composition is that it must be stable, so as not to exhibit substantial decomposition of the active ingredient during the time between manufacture of the composition and use by the patient.

It has been found that naltrexone hydrochloride may degrade upon storage, possibly due to heat, light, and/or oxygen.

At this application, naltrexone hydrochloride has a d (0.9) particle size less than 50 µm at in order to overcomes the described problems in prior art. Also, a film coated tablet is used as a pharmaceutical composition. So, this provides the desired solubility, content uniformity, compressibility of the tablet. Also, the film coated tablet has been developed by using standard techniques which is simple and cost-effective method.

### Detailed Description of the Invention

The main object of the present invention is to provide a film coated tablet comprising naltrexone hydrochloride wherein the tablet has sufficient stability during the manufacture, storage and dispensing of naltrexone hydrochloride and also the tablet has the desired dissolution profile.

Another object of the present invention is to provide a film coated tablet having improved content uniformity and compressibility.

According to this embodiment of the present invention, since naltrexone is a sensitive molecule, we found that the best oral form is a film-coated tablet. A film coated tablet is used as a pharmaceutical form for naltrexone hydrochloride. Film coating protects against the moisture and light to maintain the stability of active agent.

We have found that naltrexone hydrochloride having the following particle sizes is very important for formulation. Especially, it positively affects the dissolution properties and powder homogenization. The obtained tablets have the desired dissolution profile. The powder is more homogeneous. The content uniformity of the tablets obtained from the more homogeneous powder is more ideal. Therefore, it provides better bioavailability.

As used here in, 'particle size' means the cumulative volume size distrubition as tested by any conventionally accepted method such as the laser diffraction method (i.e. malvern analysis). The term d (0.1) means, the size at which 10% by volume of the particles are finer and d (0.5) means the size at which 50% by volume of the particles are finer and d (0.9) means the size at which %90 by volume of the particles are finer.

According to this embodiment of the present invention, a film coated tablet comprises naltrexone hydrochloride and at least one pharmaceutically acceptable excipient wherein naltrexone hydrochloride has a d (0.9) particle size less than 50 µm.

According to this embodiment of the present invention, naltrexone hydrochloride has a d (0.9) particle size less than 45 µm, preferably less than 40 µm.

According to this embodiment of the present invention, naltrexone hydrochloride has a d (0.5) particle size less than 20 µm, preferably less than 18 µm.

According to this embodiment of the present invention, naltrexone hydrochloride has a d (0.1) particle size less than 10 µm, preferably less than 8 µm.

According to this embodiment of the present invention, the amount of naltrexone hydrochloride is between 12.0% and 25.0% by weight, preferably between 15.0% and 21.0% by weight in the total film coated tablet.

According to this embodiment of the present invention, pharmaceutically acceptable excipients are selected from the group comprising fillers, binders, disintegrants, lubricants/glidants or mixtures thereof.

Suitable fillers are selected from group comprising microcrystalline cellulose, mannitol, lactose monohydrate, anhydrous lactose, sorbitol, sucrose, inorganic salts, calcium salts, spray-dried lactose, calcium silicate, polysaccharides, dextrose, sodium chloride, dextrates, lactitol, sugar pellet, starch, maltodextrin, dibasic calcium phosphate or mixtures thereof.

According to this embodiment of the present invention, the filler is microcrystalline cellulose or mannitol or mixture thereof. Using the binders helps to provide the desired dissolution profile.

According to this embodiment of the present invention, the amount of fillers is between 55.0% and 85.0% by weight, preferably between 65.0% and 80.0% by weight in the total film coated tablet.

According to this embodiment of the present invention, the amount of mannitol is between 41.0% and 60.0% by weight, preferably between 48.0% and 58.0% by weight in the total film coated tablet.

According to this embodiment of the present invention, the amount of microcrystalline cellulose is between 14.0% and 25.0% by weight, preferably between 16.0% and 20.0% by weight in the total film coated tablet.

Suitable binders are selected from the group comprising polyvinylpyrrolidone, hydroxypropyl methylcellulose, pregelatinized starch, corn starch, natural gums, gelatin, carbomers, carboxymethylcellulose sodium, cellulose acetate phthalate, chitosan, dextrose, ethylcellulose, glyceryl behenate, hydroxyethyl cellulose, hydroxyethylmethyl cellulose, hydroxypropyl cellulose, hydroxypropyl starch, magnesium aluminum silicate, poloxamer, polycarbophil, polydextrose, polyethylene oxide, polymethacrylates, aluminia hydroxide, cetostearyl alcohol, polyoxyethilene-alkyl ethers or mixtures thereof.

According to this embodiment of the present invention, the binder is polyvinylpyrrolidone.

According to this embodiment of the present invention, the amount of binders is between 1.0% and 7.0% by weight, preferably between 1.0% and 5.0% by weight in the total film coated tablet.

The selection of excipients has more importance to obtain the ideal disintegrating time during the shelf life. Especially, the choice of the disintegrant has a major role. Therefore, the choice of a suitable disintegrant and an optimal use level are critical to ensure a high disintegration rate.

Suitable disintegrants are selected from the group comprising crospovidone, povidone, croscarmellose sodium, low-substituted hydroxypropyl cellulose, sodium carboxymethyl cellulose, calcium carboxymethyl cellulose, carboxymethyl cellulose, docusate sodium, polyacrylate potassium, sodium alginate, alginic acid, magnesium aluminium silica, sulphate, poloxamer, sodium glycine carbonate or mixtures thereof.

According to this embodiment of the invention, the disintegrant is sodium starch glycolate.

According to this embodiment of the present invention, the amount of disintegrant is between 2.0% and 10.0% by weight, preferably between 3.0% and 8.0% by weight in the total film coated tablet.

Suitable lubricants/glidants are selected from the group comprising magnesium stearate, sodium stearyl fumarate, colloidal silicone dioxide, calcium stearate, zinc stearate, talc, silicon dioxide, talc, aluminium silicate, calcium silicate, magnesium silicate, magnesium oxide, starch, sodium chlorate, magnesium lauryl sulfate, sodium oleate, sodium acetate, sodium benzoate, polyethylene glycol, stearic acid, fumaric acid, glyceryl palmito sulphate, sodium lauryl sulphate or mixtures thereof.

According to this embodiment of the invention, lubricant/glidant is magnesium stearate.

The film coated tablet of the present invention can be prepared, using standard techniques and manufacturing processes well known in the art, such as direct compression, wet or dry granulation, hot melt granulation, hot melt extrusion, fluidized bed granulation, extrusion/spheronization, slugging, spray drying and solvent evaporation.

According to this embodiment of the invention, the film coated tablet of the present invention can be prepared by using wet granulation. So, an easy method was created to eliminate the disadvantages of active ingredient.

According to this embodiment of the present invention, a solvent is used at wet granulation.

Suitable solvents are selected from the group comprising pure water, dichloromethane, 0.1N HCI, methanol, ethanol, isopropyl alcohol, benzyl alcohol, propylene glycol, polyethylene glycol, glycerine, cyclomethicone, glycerine triacetate, diethylene glycol monoethyl ether or mixtures thereof. Preferably, the solvent is water.

In this present invention, a desired compressibility and a desired content uniformity of the film coated tablet is obtained and it has a simple preparation process, in favor of industrial production.

According to this embodiment of the present invention, the film coated tablet comprises;
- Naltrexone hydrochloride
- Mannitol
- Microcrystalline cellulose
- Polyvinylpyrrolidone
- Sodium starch glycolate
- Magnesium stearate.

According to this embodiment of the present invention, a process for the preparation of the film coated tablet further comprises the following steps:
a) Sieving naltrexone hydrochloride, mannitol, microcrystalline cellulose and sodium starch glycolate and then mixing into wet granulator,
b) Dissolving polyvinylpyrrolidone into water and obtained a binder solution,
c) Mixing the mixture at step (a) and the binder solution at step (b),
d) Drying wet granules,
e) Sieving the dry granules,
f) Adding magnesium stearate and then mixing,
g) Compressing the prepared mixture to form tablets,
h) Coating these tablets with film coating.

### Example 1: The film coated tablet comprising naltrexone hydrochloride

| **Agents** | **Amount (% by weight of the total tablet)** |
|---|---|
| Naltrexone hydrochloride | 12.0 - 25.0 |
| Mannitol | 41.0 - 60.0 |
| Microcrystalline cellulose | 14.0 - 25.0 |
| Polyvinylpyrrolidone | 1.0-7.0 |
| Sodium starch glycolate | 2.0 - 10.0 |
| Magnesium stearate | 0.5 - 3.0 |
| Film coating | 2.0-5.0 |
| **Total** | **100** |

### A process for example 1:

a) Sieving naltrexone hydrochloride, mannitol, microcrystalline cellulose and sodium starch glycolate and then mixing into wet granulator,
b) Dissolving polyvinylpyrrolidone into water and obtained a binder solution,
c) Mixing the mixture at step (a) and the binder solution at step (b),
d) Drying wet granules,
e) Sieving the dry granules,
f) Adding magnesium stearate and then mixing,
g) Compressing the prepared mixture to form tablets,
h) Coating these tablets with film coating.

## Claims

1. A film coated tablet comprising naltrexone hydrochloride and at least one pharmaceutically acceptable excipient wherein naltrexone hydrochloride has a d (0.9) particle size less than 50 µm.

2. The film coated tablet according to claim 1, wherein naltrexone hydrochloride has a d (0.5) particle size less than 20 µm, preferably less than 18 µm.

3. The film coated tablet according to claim 1, wherein naltrexone hydrochloride has a d (0.1) particle size less than 10 µm, preferably less than 8 µm.

4. The film coated tablet according to claim 1, wherein at least one pharmaceutically acceptable excipient is selected from the group comprising fillers, binders, disintegrants, lubricants/glidants or mixtures thereof.

5. The film coated tablet according to claim 4, wherein fillers are selected from group comprising microcrystalline cellulose, mannitol, lactose monohydrate, anhydrous lactose, sorbitol, sucrose, inorganic salts, calcium salts, spray-dried lactose, calcium silicate, polysaccharides, dextrose, sodium chloride, dextrates, lactitol, sugar pellet, starch, maltodextrin, dibasic calcium phosphate or mixtures thereof.

6. The film coated tablet according to claim 5, wherein the filler is microcrystalline cellulose or mannitol or mixture thereof.

7. The film coated tablet according to claim 4, wherein binders are selected from the group comprising polyvinylpyrrolidone, hydroxypropyl methylcellulose, pregelatinized starch, corn starch, natural gums, gelatin, carbomers, carboxymethylcellulose sodium, cellulose acetate phthalate, chitosan, dextrose, ethylcellulose, glyceryl behenate, hydroxyethyl cellulose, hydroxyethylmethyl cellulose, hydroxypropyl cellulose, hydroxypropyl starch, magnesium aluminum silicate, poloxamer, polycarbophil, polydextrose, polyethylene oxide, polymethacrylates, aluminia hydroxide, cetostearyl alcohol, polyoxyethilene-alkyl ethers or mixtures thereof.

8. The film coated tablet according to claim 7, wherein the binder is polyvinylpyrrolidone.

9. The film coated tablet according to claim 4, wherein disintegrants are selected from the group comprising crospovidone, povidone, croscarmellose sodium, low-substituted hydroxypropyl cellulose, sodium carboxymethyl cellulose, calcium carboxymethyl cellulose, carboxymethyl cellulose, docusate sodium, polyacrylate potassium, sodium alginate, alginic acid, magnesium aluminium silica, sulphate, poloxamer, sodium glycine carbonate or mixtures thereof.

10. The film coated tablet according to claim 9, wherein the disintegrant is sodium starch glycolate.

11. The film coated tablet according to claim 4, wherein lubricants/glidants are selected from the group comprising magnesium stearate, sodium stearyl fumarate, colloidal silicone dioxide, calcium stearate, zinc stearate, talc, silicon dioxide, talc, aluminium silicate, calcium silicate, magnesium silicate, magnesium oxide, starch, sodium chlorate, magnesium lauryl sulfate, sodium oleate, sodium acetate, sodium benzoate, polyethylene glycol, stearic acid, fumaric acid, glyceryl palmito sulphate, sodium lauryl sulphate or mixtures thereof.

12. The film coated tablet according to claim 1, wherein the film coated tablet is prepared by using wet granulation.

13. The film coated tablet according to claim 1, comprising;
- Naltrexone hydrochloride
- Mannitol
- Microcrystalline cellulose
- Polyvinylpyrrolidone
- Sodium starch glycolate
- Magnesium stearate.

14. A process for the preparation of the film coated tablet comprising the following steps:
a) Sieving naltrexone hydrochloride, mannitol, microcrystalline cellulose and sodium starch glycolate and then mixing into wet granulator,
b) Dissolving polyvinylpyrrolidone into water and obtained a binder solution,
c) Mixing the mixture at step (a) and the binder solution at step (b),
d) Drying wet granules,
e) Sieving the dry granules,
f) Adding magnesium stearate and then mixing,
g) Compressing the prepared mixture to form tablets,
h) Coating these tablets with film coating.
